# EUROPEAN PATENT SPECIFICATION

(11) **EP 2 381 968 B1**
(45) Date of publication and mention of the grant of the patent: **09.05.2018**
(21) Application number: 09836582.8
(22) Date of filing: 06.11.2009
(51) Int. Cl.: A61L 15/32, A61L 15/28, A61K 9/70, A61K 9/00, A61K 47/42, A61F 13/00, A61L 15/14

(54) **BIOPOLYMERIC MEMBRANE FOR WOUND PROTECTION AND REPAIR**
BIOPOLYMER-MEMBRAN FÜR WUNDSCHUTZ UND -REPARATUR
MEMBRANE BIOPOLYMÈRE POUR PROTECTION ET RÉPARATION DE PLAIE

(30) Priority: 30.12.2008 US 346482
(43) Date of publication of application: 02.11.2011
(73) Proprietor: Collagen Matrix, Inc., Franklin Lakes, NJ 07869 (US)
(72) Inventor: LI, Shu-Tung, Oakland, New Jersey 07436 (US); LEE, Natsuyo Shishido, Bridgewater, New Jersey 08807 (US); YUEN, Debbie, Woodcliff Lake, New Jersey 07675 (US)
(74) Representative: Chajmowicz, Marion
(86) International application number: PCT/US2009/063549
(87) International publication number: WO 2010/077434

(56) References cited:
- EP-A1- 1 016 757
- WO-A1-93/11803
- WO-A2-2006/047496
- US-A- 5 951 535
- US-B2- 7 374 777
- US-B2- 7 374 777

## Description

### Background of the Invention

Surgical procedures cause wounds at the target sites. For example, neural surgery often results in wounds at the dura mater, i.e., the membrane covering the brain and spinal cord. Biopolymeric membranes facilitate wound healing and protect wounds from invasion of fibrotic cells.

The document WO2006/047496 teaches a sheet biopolymeric membrane for repairing damaged tissue.

The document US7374777 teaches a sheet biopolymeric membrane having oriented fibers.

### Summary of the Invention

The present invention features a biopolymeric membrane suitable for repairing wounds. A biocompatible membrane for tissue repair, comprising: a first layer including randomly oriented fibers of a first biopolymer that is collagen, the fibers of the first biopolymer having a fiber length ranging from 10 cm to 40 cm, and a second layer including fibers of a second biopolymer that is collagen, the fibers of the second biopolymer having a fiber length ranging from 0.1 mm to 1 mm, wherein the second layer has a thickness ranging from 1 µm to 50 µm, wherein said biocompatible membrane is permeable to molecules with a molecular weight of less than 29,000 daltons, has a hydrothermal shrinkage temperature of 50-70 °C, a draping angle of 45 to 90 degrees, a density of 0.20 to 0.40 g/cm³, and has a thickness ranging from 0.3 mm to 0.7 mm, the fibers of first biopolymer and the fibers of second biopolymer being cross-linked together. It can further contain one or more bioactive agents, e.g., various growth factors and antibiotics.

This invention also features a method of making the biopolymeric membrane described above. The method includes: dispersing fibers of a first biopolymer that is collagen in an acidic or alkaline solution to form a mixture,neutralizing the mixture to a pH equal to the isoelectric point of the first biopolymer to produce reconstituted fibers of the first biopolymer having a fiber length ranging from 2.5 cm to 50 cm,dehydrating the reconstituted fibers, compressing the dehydrated fibers to form a first layer, in which the reconstituted fibers are randomly oriented,freeze-drying the first layer, coating the freeze-dried first layer with fibers of a second biopolymer that is collagen; to form a membrane that has a thickness ranging from 0.3 mm to 0.7 mm and a density ranging from 0.2 to 0.4 g/cm³, the fibers of the second biopolymer having a fiber length of 0.1 mm to 1 mm and forming a second layer on top of the first layer, wherein the second layer has a thickness ranging from 1 µm to 50 µm, and crosslinking the fibers of the first biopolymer and the fibers of the second biopolymer in the membrane. The collagen fibers are dispersed in a solution having a pH value either lower than 3 or higher than 11 to form a mixture. The mixture is then neutralized to a pH of 4.5-6 (e.g., 5.0-5.5) to produce reconstituted collagen fibers.

The details of one or more embodiments of the invention are set forth in the description below. Other features or advantages of the present invention will be apparent from the following detailed description of several embodiments, and also from the appended claims.

### Detailed Description of the Invention

The present invention relates to a bioresorbable, conformable, and semi-permeable biopolymeric membrane for tissue protection and repair, e.g., for repairing wounds at the dura mater in patients who have undergone neural surgery.

The biopolymeric membrane of this invention has two layers as described in claim 1. The first layer contains randomly oriented fibers made from collagen The fibers used for making this layer are reconstituted so that they are longer than their un-reconstituted counterparts. The second layer, i.e., the coating layer, is formed by coating the first layer with fibers made from collagen. This layer is very thin, i.e., having a thickness of only 1-50 µm (e.g., 3-20 µm).

The two-layer biopolymer membrane described above can further contain one or more bioactive agents, e.g., growth factors and antibiotics. The bioactive agent(s) can form an additional layer either in between the first and second layers described above, or on the surface of the membrane. Alternatively, the bioactive agent(s) is interspersed in the first or second layer.

The biopolymeric membrane of this invention possesses the following features.

First, it is highly conformable, i.e., having a draping angle ranging from 45 to 90 degree. The draping angle of a membrane is determined as follows. Fix one half of a membrane onto a horizontal surface and allow the other half to drape via gravity. The angle between the draped half and the horizontal surface is assigned as the draping angle of the membrane. Possessing a high conformability, the biopolymeric membrane of this invention can be used to substantially seal a wound site that has an irregular shape and a non-smooth contour.

Second, the biopolymeric membrane of this invention is semi-permeable, i.e., permeable only to molecules having a molecule weight less than 29,000 daltons. As such, this membrane protects a wound site from cell invasion but allows passage of small nutrient molecules through it.

Third, the membrane is stable in vivo; namely, it has a hydrothermal shrinkage temperature in the range of 50 to 70°C (corresponding to an *in vivo* resorption time of 2-12 months).

It is preferred that the membrane possesses a suture pullout strength of 0.1-0.7 kg (e.g., 0.15-0.5 kg), a tensile strength of 5-100 kg/cm² (e.g., 20-50 kg/cm²), and a density of 0.20 to 0.40 g/cm³(e.g., 0.04 - 0.3 g/cm³).

Given the above-described features, the biopolymeric membrane of this invention is suitable for facilitating wound healing and occluding cell invasion. Particularly, this biopolymeric membrane is useful as an onlay graft for dura repair.

The biopolymer membrane of this invention can be prepared as follows:
Fibers made from the first biopolymer that is collagen are dispersed in an acidic (e.g., pH < 3) or alkaline (e.g., pH >11) solution to form a mixture. The biopolymer fibers can be either isolated from their natural sources or synthesized chemically. Suitable acidic solutions include solutions prepared with an organic acid, e.g., acetic acid and lactic acid, or with an inorganic acid, e.g., hydrochloric acid and sulfuric acid. When an alkaline solution is used, it can be prepared using sodium hydroxide, potassium hydroxide, or calcium hydroxide.

Next, the mixture is neutralized to a pH value equal to the isoelectric point of the first biopolymer, i.e., within 20% of the isoelectric point of that biopolymer. At this pH, the biopolymer fibers mentioned above reconstitute to form fibers having a length greater than 5 cm. The reconstituted fibers, separated from the liquid phase of the mixture, are then partially dehydrated to remove the liquid using a meshed screen or a similar device via a mechanical force. The extent of this partial dehydration determines the conformability and density of the membrane to be made. The reconstituted fibers are then compressed to form a sheet with desired thickness, size, and wet weight. These factors also determine the density of the resultant membrane. The sheet can then freeze dried to form the first layer with a Virtis freeze dryer via methods known in the art. See, e.g., US Patent 5,026,381.

Subsequently, the first layer is coated by spraying on it a dispersion containing fibers made from a second biopolymer, which can be prepared by the method described above. The biopolymer fibers contained in this dispersion has a low content and a short fiber length so that they can form mist particles during spraying without blocking the nozzle of the spray apparatus. The spraying step can be performed using any spray apparatus that can deposit mist-like polymer particles onto the surface of the first layer without affecting its biomechanical characteristics. Suitable spray apparatuses include commercially available sprayers, air brushes, or custom-designed sprayers. Upon spraying, the fibers of the second biopolymer form a coating layer (i.e., the second layer) on the first layer to produce a two-layer membrane. The thickness of the coating layer, ranging from about 1 µm to about 50 µm, controls permeability. When necessary, the coating step can be repeated so as to achieve the desired thickness and permeability.

Finally, the two-layer membrane described above is cross-linked to affix the biopolymer fibers, thus producing a biopolymeric membrane of this invention. The fibers can be cross-linked by a chemical cross-linking agent (e.g., an aldehyde compound or a carbodimiide). Formaldehyde is a preferred cross-linking agent, given its high vapor pressure and its safe use in implantable devices. Alternatively, the fibers can also be cross-linked by heat and vacuum, ultraviolet, or low-dose gamma irradiation.

When a cross-linking agent is used, the extent of the cross-linking can be controlled by the concentration of the cross-linking agent, the time for the cross-linking reaction, and the temperature (if the agent is in liquid form), or the vapor pressure (if the agent is in gas form), under which the cross-linking reaction is taken. The extent of the cross-linking controls the *in vivo* resorption time of the membrane.

If desired, one or more bioactive agents can be incorporated into the biopolymeric membrane described herein. The bioactive agent(s) can be mixed with fibers of the second biopolymer and interspersed into the second layer, or is sprayed (for agents in liquid form) or dusted (for agents in powder form) on the second layer. Alternatively, it is mixed with the fibers of the first biopolymer and interspersed into the first layer, or is sprayed/dusted on the first layer prior to the formation of the second layer described above. The first layer is then covered with the second layer on the surface where the bioactive agent(s) stays to form a sandwich-structured membrane having the bioactive agent(s) in between the first and second layer. The thickness of the second layer controls the release rate of the bioactive agent(s). The release rate is also controlled by factors such as the agent's particle size, hydrophilicity, and interaction with the biopolymer fibers included in either the first or the second layer.

Below is an example of making the biopolymeric membrane of this invention, using collagen. Collagen fibers are prepared from collagen rich tissues, e.g., skin, bone, tendon, and ligament, via methods known in the art. See, e.g., US Patent 5,512,291; and Oneson, et al., J. Am. Leather Chemists Assoc. 65:440-450, 1970. They are then dispersed in an acidic solution to form a collagen dispersion. See US Patents 3,157,542 and 5,326,350. Alternatively, the collagen dispersion can be prepared by mixing collagen fibers in an alkaline solution. The preferable collagen content in the collagen dispersion ranges from 0.5 % to 1.5% by weight. Normally, the length of the collagen fibers in the dispersion thus prepared is less than 3 mm.

The collagen dispersion is then neutralized to a pH value of 4.5-6 (e.g., 5). At this pH, the collagen fibers reconstitute to form long collagen fibers having a fiber length of 10-40 cm. The reconstituted collagen fibers are then partially dehydrated and subsequently compressed to form a collagen sheet. After being freeze dried, the collagen sheet is coated via spray with a collagen dispersion containing unreconstituted collagen fibers to produce a collagen membrane. In this dispersion, the collagen content is preferably less than 0.1% and the collagen fibers have a length less than 1 mm. The collagen membrane is then treated with formaldehyde to cross link the collagen fibers contained in the membrane.

Without further elaboration, it is believed that one skilled in the art can, based on the above description, utilize the present invention to its fullest extent.

### Example 1. Preparation and Characterization of a Conformable and Semi-Permeable Collagen Membrane

### Preparation of purified collagen fibers

The fat and fascia of bovine flexor tendon were carefully removed and washed with water. The cleaned tendon was frozen and comminuted by slicing into 0.5 mm slices with a meat slicer. One kilogram of sliced wet tendon was first extracted in 5 liters of distilled water at room temperature for 24 hours. The extractant was discarded and 5 liters of 0.2 M HCl in 0.5 M Na₂SO₄ was added and the tendon slices were extracted at room temperature for 24 hours. The acid solution was discarded and 5 liters of 0.5 M Na₂SO₄ was added to wash the tendon and to remove the residual acid. The acid extracted tendon was then extracted in 5 liters of 0.75 M NaOH in the presence of 1 M Na₂SO₄ at room temperature for 24 hours. The base solution was then discarded. The residual base was neutralized with 3 M HCl to pH 5 followed by several changes of distilled water to remove the residual salts associated with the tendon. The tendon was then defatted with isopropanol (tendon: isopropanol = 1:5, v/v) for 24, 46, and 6 hours at 25°C under constant agitation. The extractant was decanted and an equal volume of isopropanol was added and the tendon slices were extracted overnight at 25°C under constant agitation. The defatted tendon was then dried under a clean hood. The purified collagen fibers were stored dry at room temperature until further processing.

### Preparation of collagen fiber dispersions

Purified collagen fibers were weighed and dispersed in 0.07 M lactic acid, homogenized with a Silverson Homogenizer (East Longmeadow, MA), and then filtered with a stainless steel mesh filter (30 mesh). The dispersion, which had a collagen content of 0.7% (w/v), was de-aerated with vacuum to remove the trapped air.

Alternatively, purified collagen fibers were weighed and dispersed in 0.005 M NaOH, homogenized with a Silverson Homogenizer (East Longmeadow, MA), and then filtered with a stainless steel mesh filter (40 mesh). The dispersion, which had a collagen content of 1.0% (w/v), was deaerated with vacuum to remove the air trapped in it.

### Reconstitution of Collagen Fibers

Acid dispersed collagen fibers (180g) were mixed with 20 ml 0.6% NH₄OH to form a solution having a pH value of 4.7-5.0 (i.e., the isoelectric point of collagen) to produce reconstitute collagen fibers. The reconstituted collagen fibers have a fiber length of 2.5-50 cm.

### Fabrication of a Conformable and Semi-Permeable Collagen Membrane

The reconstituted collagen fibers described above were partially dehydrated using a stainless steel screen by gently squeezing the water out from the wet fibers to a weight that gives a density in the range of 0.2 to 0.25 g of collagen/cm³. The partially dehydrated fibers were then compressed into a sheet membrane using a roller. The membrane was freeze-dried (10°C for 24 hours, 20°C for 16 hours at a pressure less than 200 millitorr) using a Virtis Freeze Dryer (Gardiner, N.Y.). The dried membrane of collagen fibers was hung vertically in a chamber and sprayed with 0.05% (w/v) lactic acid dispersed collagen fibers. Care was taken to ensure that the surface was uniformly coated with a thin layer of collagen fibers. The collagen-fiber coated membrane was dried in air and then crosslinked with formaldehyde vapor, generated from 0.1% formaldehyde solution at ambient temperature, for 6 hours.

### Characterization of the Conformable and Semipermeable Collagen Membrane

Physical, chemical, and mechanical characteristics of the membranes described in the proceeding example were assessed in the following ways:

### i) Thickness

The thickness of the membranes was determined with a caliper.

### ii) Density

The density (g/cm³) of a membrane was determined as follows. The membrane was first dried under vacuum for 24 hours or over P₂O₅ for 24 hours and the dry weight recorded. The length, width, and thickness of the membrane were then measured with a caliper. The density is calculated using the formula: (weight of the membrane)/(volume of the membrane).

### iii) Hydrothermal Shrinkage Temperature (Tₛ)

A circular sample of the membrane was punched, hydrated in a phosphate buffer with a concentration of 0.01M, and a pH of 7.4, sealed in an aluminum cell, placed in a differential scanning calorimeter (DSC, Metter-Toledo, Inc., Columbus OH), and heated at a rate of 5°C/min. The temperature, at which the collagen in the membrane started losing its triple helical structure, was recorded as the hydrothermal shrinkage temperature.

### iv) Suture pullout strength

The suture pullout strength of the membrane was determined with a tensile tester (Chatillon, Greensboro, NC) as follows. The membrane was cut to a size of 20 mm x 15 mm and soaked in a phosphate buffered saline solution (pH 7.4) at 25°C for about 2 minutes. A suture (3-0 silk black braided, taper SH-1, Ethicon, Somerville, NJ) was placed through the membrane at a position approximately 4 mm from the 20 mm edge. One end of the suture was tied into a knot and the other end of the suture was secured to a hook adapter of the tensile tester. After the sample was fixed onto a clamp, the suture was pulled at a speed of 2.5 cm/min until it was pulled out. The strength to pull out the suture (i.e., the suture pullout strength) was recorded by the tensile tester.

### v) Tensile strength

Tensile strength of the membrane was also determined by the tensile tester described above. The membrane was cut into a dumbbell shape with a die punch and soaked in a phosphate buffered saline solution (pH 7.4) at 25°C for about 2 minutes. The sample was then secured onto a clamp fixture, and pulled at a speed 2.5 cm/min until the sample was pulled apart. The strength under which the membrane was pulled apart was recorded as the tensile strength.

### vi) Permeability

A 2-cm diameter disk cut from the membrane was inserted into a hole between two compartments (i.e., compartment 1 and compartment 2) of a chamber, thereby completely separating the two compartments. A fixed volume of PBS containing 50 µg carbonic anhydrase (having a molecule weight of 29,000 dalton) per ml was added to compartment 1 and a fixed volume of PBS was added to compartment 2. After equilibrated for 24 hours, the PBS in compartment 2 was determined for the percent of carbonic anhydrase contained therein by the carbonic anhydrase assay system described in Li, S.T., et. al., Biotechnology and Polymers:281-293, 1991.

### vii) Surface Characteristics

Surface morphology (i.e., macroscopic and microscopic structures of the membrane surface) was determined with a scanning electron microscope (SEM) at various magnifications.

Certain characteristics (average± standard deviation) of the biopolymeric membrane described herein are summarized below:

| Density (g/cm³) [6] | Thickness of the layer made from reconstituted collagen fibers (mm) [20] | Thickness of the coating layer (µm) [6] | Percent of permeated carbonic anhydrase in 24 hrs [6] | Hydrothermal shrinkage temp. (°C) [13] | Suture pull-out strength (kg) [6] | Tensile strength (kg/cm²) [6] | Surface Morphology |
|---|---|---|---|---|---|---|---|
| 0.30±0.06 | 0.43±0.08 | 3.7±0.4 | 7.8±2.1 | 52±1.0 | 0.21±0.01 | 28.5±6.8 | Smooth |

| | | | | | | | |
|---|---|---|---|---|---|---|---|
| * The number in [] represents the number of samples tested | | | | | | | |

### Example 2. Use of a Conformable and Semipermeable Collagen Membrane in Neural Surgery for Dura Repair and Regeneration

The collagen membrane prepared by the method described in Example 1 is used to promote dura repair and regeneration in a rabbit model. New Zealand white rabbits (3 to 4 kg) are anesthetized using xylazine (5 mg/kg) and ketamine (35 mg/kg) via intramuscular injection and maintained sedated using halothane (0.5 to 2%) via endotracheal tube. Their scalps are shaved and washed with Betadine. Under aseptic conditions, the scalps are incised coronally to expose the perioseum. The periosteum is then stripped from the calvarium using a periosteal elevator. Two medial and 2 lateral 2.1 mm burr holes are placed to avoid the sagittal and transverse venous sinuses and orbital cavity. Bone wax and electrocautery are used to control bleeding. A Dremel motor tool is used to cut a trapezoid-shaped craniotomy and elevate the bone flap hinged on the pericranium and the muscles attached to it. The bone flaps are removed with care to avoid damage to the underlying meninges and cerebral cortex. Using a dural hook, the dura mater is gently lifted and incised. Angled irridectomy scissors are used to create an 8 mm x 8 mm damaged area in the dura mater. The collagen membrane, cut into a size of 1 cm², is soaked in sterile saline for 5 minutes and placed over the damaged area (1mm overlap on dura) without suturing. The bone flaps are replaced and the periosteum is closed with a 3-0 chromic gut while the scalps are closed with a 2-0 vicryl suture.

## Claims

1. A biocompatible membrane for tissue repair, comprising:
a first layer including randomly oriented fibers of a first biopolymer that is collagen, the fibers of the first biopolymer having a fiber length ranging from 10 cm to 40 cm, and
a second layer including fibers of a second biopolymer that is collagen, the fibers of the second biopolymer having a fiber length ranging from 0.1 mm to 1 mm, wherein the second layer has a thickness ranging from 1 µm to 50 µm,
wherein said biocompatible membrane is permeable to molecules with a molecular weight of less than 29,000 daltons, has a hydrothermal shrinkage temperature of 50-70 °C, a draping angle of 45 to 90 degrees, a density of 0.20 to 0.40 g/cm³, and has a thickness ranging from 0.3 mm to 0.7 mm, the fibers of first biopolymer and the fibers of second biopolymer being cross-linked together.

2. The biocompatible membrane of claim 1, further comprising a bioactive agent.

3. A method of making a biopolymer membrane for tissue repair, comprising:
dispersing fibers of a first biopolymer that is collagen in an acidic or alkaline solution to form a mixture,
neutralizing the mixture to a pH equal to the isoelectric point of the first biopolymer to produce reconstituted fibers of the first biopolymer having a fiber length ranging from 2.5 cm to 50 cm,
dehydrating the reconstituted fibers,
compressing the dehydrated fibers to form a first layer, in which the reconstituted fibers are randomly oriented,
freeze-drying the first layer,
coating the freeze-dried first layer with fibers of a second biopolymer that is collagen to form a membrane that has a thickness ranging from 0.3 mm to 0.7 mm and a density ranging from 0.2 to 0.4 g/cm³, the fibers of the second biopolymer having a fiber length of 0.1 mm to 1 mm and forming a second layer on top of the first layer, wherein the second layer has a thickness ranging from 1 µm to 50 µm, and
crosslinking the fibers of the first biopolymer and the fibers of the second biopolymer in the membrane.

4. The method of claim 3, wherein the fibers of the first biopolymer are dispersed in the solution having a pH value lower than 3 to form the mixture, and the mixture is then neutralized to a pH ranging from 4.5 to 6 to produce reconstituted fibers of collagen.

5. The method of claim 3, wherein the coating step is performed by spraying on the first layer a dispersion containing the fibers of the second polymers.

6. The biocompatible membrane of claim 1, wherein the second layer a thickness ranging from 3 µm to 20 µm.

## Patentansprüche

1. Eine biokompatible Membran zur Gewebereparatur, umfassend:
eine erste Schicht, die zufällig orientierte Fasern eines ersten Biopolymers einschließt, das Kollagen ist, wobei die Fasern des ersten Biopolymers eine Faserlänge im Bereich von 10 cm bis 40 cm haben, und
eine zweite Schicht, die Fasern eines zweiten Biopolymers einschließt, das Kollagen ist, wobei die Fasern des zweiten Biopolymers eine Faserlänge im Bereich von 0,1 mm bis 1 mm haben, wobei die zweite Schicht eine Dicke im Bereich von 1 µm bis 50µm hat,
wobei die biokompatible Membran für Moleküle mit einem Molekulargewicht von weniger als 29.000 Daltons durchlässig ist, eine hydrothermale Schrumpfungstemperatur von 50-70 °C, einen Drapierwinkel von 45 bis 90 Grad, eine Dichte von 0,20 bis 0,40 g/cm³, und eine Dicke im Bereich von 0,3 mm bis 0,7 mm hat, und wobei die Fasern des ersten Biopolymers und die Fasern des zweiten Biopolymers miteinander quervernetzt sind.

2. Die biokompatible Membran nach Anspruch 1, ferner umfassend einen bioaktiven Wirkstoff.

3. Ein Verfahren zur Herstellung einer Biopolymermembran zur Gewebereparatur, umfassend:
Dispergieren von Fasern eines ersten Biopolymers, das Kollagen ist, in einer sauren oder basischen Lösung, um ein Gemisch zu bilden,
Neutralisieren des Gemischs auf einen pH, der dem isoelektrischen Punkt des ersten Biopolymers entspricht, um rekonstituierte Fasern des ersten Biopolymers mit einer Faserlänge im Bereich von 2,5 cm bis 50 cm herzustellen,
Dehydrieren der rekonstituierten Fasern,
Komprimieren der dehydrierten Fasern, um eine erste Schicht zu bilden, in der die rekonstituierten Fasern zufällig orientiert sind,
Gefriertrocknen der ersten Schicht,
Beschichten der gefriergetrockneten ersten Schicht mit Fasern eines zweiten Biopolymers, das Kollagen ist, um eine Membran zu bilden, die eine Dicke im Bereich von 0,3 mm bis 0,7 mm und eine Dichte im Bereich von 0,2 bis 0,4 g/cm³hat, die Fasern des zweiten Biopolymers eine Faserlänge von 0,1 mm bis 1 mm haben und eine zweite Schicht auf der ersten Schicht bilden, wobei die zweite Schicht eine Dicke im Bereich von 1 µm bis 50 µm hat, und
Quervernetzen der Fasern des ersten Biopolymers und der Fasern des zweiten Biopolymers in der Membran.

4. Das Verfahren nach Anspruch 3, wobei die Fasern des ersten Biopolymers in der Lösung mit einem pH-Wert von weniger als 3 dispergiert werden, um ein Gemisch zu bilden, und das Gemisch dann auf einem pH im Bereich von 4,5 bis 6 neutralisiert wird, um rekonstituierte Kollagenfasern herzustellen.

5. Das Verfahren nach Anspruch 3, wobei der Beschichtungsschritt mittels Aufsprühen einer Dispersion, die Fasern des zweiten Polymers enthält, auf die erste Schicht durchgeführt wird.

6. Die biokompatible Membran nach Anspruch 1, wobei die zweite Schicht eine Dicke im Bereich von 3 µm bis 20 µm hat.

## Revendications

1. Membrane biocompatible pour réparation tissulaire comprenant :
une première couche comprenant des fibres orientées au hasard d'un premier biopolymère qui est du collagène, les fibres du premier biopolymère ayant une longueur de fibre située dans la plage allant de 10 cm à 40 cm, et
une deuxième couche contenant des fibres d'un deuxième biopolymère qui est du collagène, les fibres du deuxième biopolymère ayant une longueur de fibre située dans la plage allant de 0,1 mm à 1 mm, laquelle deuxième couche a une épaisseur située dans la plage allant de 1 µm à 50 µm,
laquelle membrane biocompatible est perméable aux molécules ayant un poids moléculaire inférieur à 29 000 daltons, a une température de rétraction hydrothermique de 50 à 70 °C, un angle de drapage de 45 à 90 degrés, une masse volumique de 0,20 à 0,40 g/cm³, et a une épaisseur située dans la plage allant de 0,3 mm à 0,7 mm, les fibres du premier biopolymère et les fibres du deuxième biopolymère étant réticulées ensemble.

2. Membrane biocompatible selon la revendication 1, comprenant en outre un agent bioactif.

3. Méthode de préparation d'une membrane biopolymère pour réparation tissulaire, comprenant :
la dispersion de fibres d'un premier biopolymère qui est du collagène dans une solution acide ou alcaline pour former un mélange,
la neutralisation du mélange à un pH égal au point isoélectrique du premier biopolymère pour produire des fibres reconstituées du premier biopolymère ayant une longueur de fibre située dans la plage allant de 2,5 cm à 50 cm,
la déshydratation des fibres reconstituées,
la compression des fibres déshydratées pour former une première couche dans laquelle les fibres reconstituées sont orientées au hasard,
la lyophilisation de la première couche,
le revêtement de la première couche lyophilisée par des fibres d'un deuxième biopolymère qui est du collagène, pour former une membrane qui a une épaisseur située dans la plage allant de 0,3 mm à 0,7 mm et une masse volumique située dans la plage allant de 0,2 à 0,4 g/cm³, les fibres du deuxième biopolymère ayant une longueur de fibre de 0,1 mm à 1 mm et formant une deuxième couche au-dessus de la première couche, laquelle deuxième couche a une épaisseur située dans la plage allant de 1 µm à 50 µm, et
la réticulation des fibres du premier biopolymère et des fibres du deuxième biopolymère dans la membrane.

4. Méthode selon la revendication 3, dans laquelle les fibres du premier biopolymère sont dispersées dans la solution ayant une valeur de pH inférieure à 3 pour former le mélange, et le mélange est ensuite neutralisé à un pH situé dans la plage allant de 4,5 à 6 pour produire des fibres reconstituées de collagène.

5. Méthode selon la revendication 3, dans laquelle l'étape de revêtement est effectuée par pulvérisation sur la première couche d'une dispersion contenant les fibres du deuxième polymère.

6. Membrane biocompatible selon la revendication 1, dans laquelle la deuxième couche a une épaisseur située dans la plage allant de 3 µm à 20 µm.
